(19) **Europäisches Patentamt / European Patent Office / Office européen des brevets**

(11) **EP 2 214 015 B2**

(12) **NEW EUROPEAN PATENT SPECIFICATION**

After opposition procedure

(45) Date of publication and mention
of the opposition decision:
**27.12.2023 Bulletin 2023/52**

(45) Mention of the grant of the patent:
**02.11.2011 Bulletin 2011/44**

(21) Application number: **10158529.7**

(22) Date of filing: **19.04.2002**

(51) International Patent Classification (IPC):
*G01N 33/53* (2006.01)      *G01N 33/543* (2006.01)
*G01N 33/537* (2006.01)      *B01L 3/00* (2006.01)

(52) Cooperative Patent Classification (CPC):
**G01N 33/54393; B01L 3/502746; G01N 33/52;**
B01L 2200/12; B01L 2300/046; B01L 2300/0654;
B01L 2300/0825; B01L 2300/0829;
B01L 2300/0887; B01L 2300/161

(54) **Hydrophilic diagnostic devices for use in the assaying of biological fluids**

Hydrophile Diagnosevorrichtungen zur Verwendung beim Testen biologischer Flüssigkeiten

Dispositifs diagnostiques hydrophiles utilisés dans l'analyse de fluides biologiques

(84) Designated Contracting States:
**DE FR GB IE IT**

(30) Priority: **19.04.2001 US 284527 P**

(43) Date of publication of application:
**04.08.2010 Bulletin 2010/31**

(62) Document number(s) of the earlier application(s) in
accordance with Art. 76 EPC:
**02728841.4 / 1 390 750**

(73) Proprietor: **ADHESIVES RESEARCH, INC.**
**Glen Rock,**
**PA 17327 (US)**

(72) Inventors:
• **Meathrel, William G.**
**York, PA 17404 (US)**
• **Hand, Herbert M., Sr.**
**Bel Air, MD 21014 (US)**
• **Su, Li-Hung**
**Loganville, PA 17342 (US)**

(74) Representative: **Beck Greener LLP**
**Fulwood House**
**12 Fulwood Place**
**London WC1V 6HR (GB)**

(56) References cited:
EP-A- 0 408 378        WO-A-98/39645
WO-A1-99/58245        WO-A2-02/04570
DE-A1- 3 903 126       US-A- 5 759 364
US-A- 6 121 508

• **P. GRODZINSKI ET AL: "Development of plastic microfluidic devices for sample preparation" BIOMEDICAL MICRODEVICES, vol. 3, no. 4, December 2001 (2001-12), pages 275-283, XP002585080 ISSN: 1387-2176 DOI: 10.1023/A:1012400629650**
• **JAMES LEE L ET AL: "DESIGN AND FABRICATION OF CD-LIKE MICROFLUIDIC PLATFORMS FOR DIAGNOSTICS: POLYMER-BASED MICROFABRICATION" BIOMEDICAL MICRODEVICES, KLUWER, DORDRECHT, NL LNKD-DOI:10.1023/A:1012469017354, vol. 3, no. 4, 1 January 2001 (2001-01-01), pages 339-351, XP009015637 ISSN: 1387-2176**

EP 2 214 015 B2

**Description**

**Background of the Invention**

This application is directed to a method of manufacturing microfluidic in-vitro diagnostic devices.

Microfluidic devices are commonly-used in the assaying of biological samples. Such devices comprise a base platform within which are formed a number of capillaries which serve to transport the sample from a receiving portion of the device to a collection portion.

In-vitro diagnostic devices are used to detect analytes such as nutrients, hormones, therapeutic drugs, drugs-of-abuse and environmental contaminates. In medical diagnostic test devices, biological fluids such as whole blood, plasma, serum, nasal secretions, sputum, saliva, urine, sweat, transdermal exudates or cerebrospinal fluids may be analyzed for specific components that are clinically important for monitoring and diagnosis. In addition, microbiological suspensions and tissues may be homogenized in compatible liquids and the fluid analyzed for specific components. Typically, the specimen fluid is deposited at an inlet port of a suitable in-vitro diagnostic test strip and the sample fluid is drawn into the device by mechanical means such as vacuum or by capillary flow action.

In-vitro diagnostic devices are used in various settings including hospitals, clinics, alternative care sites and in the home. These devices have been developed by various manufacturers to enable clinical professionals and non-professionals to make accurate decisions for the diagnosis and management of medical conditions. Point-of-care devices such are used to analyze blood chemistry such as electrolytes and pH in both clinical and non-clinical locations. Home pregnancy test kits are used to monitor hcG in urine. Diabetics routinely use diagnostic test strips to monitor blood glucose concentrations. Amira Medical, "Glucose Monitor without Fingersticking", IVD Technology, July 1999, p. 16.

Various types of capillary flow type diagnostic devices are known and have been used for some time. Exemplary of such devices are those shown in U.S. Patent Nos. 6,048,498 *and* 6,117,395.

**Objects and Summary of the Invention**

It is an object of the present invention to provide diagnostic devices which enable benefits to be achieved not achieved by prior art devices.

It is an object of the present invention to provide a microfluidic device which provides uniform wetting and wicking, ease of manufacture, which is non-contaminating to the sample, exhibits controlled evaporation and enables separation of components to be achieved.

In accordance with one aspect of the invention, there is provided a method of manufacturing a microfluidic in-vitro diagnostic device, comprising providing a base having at least one fluid channel within which a fluid sample to be assayed passes from an inlet port to a detection zone and enclosing said at least one fluid channel with at least one enclosure surface, wherein said at least one enclosure surface of the fluid channel comprises a hydrophilic heat-sealable adhesive or a hydrophilic pressure-sensitive adhesive and is hydrophilic in character to increase the surface energy of the fluid flow path to enhance the flow of biological fluids in the channel.

In accordance with still another embodiment of the invention, there is provided a microfluidic in-vitro diagnostic device comprised of opposing base portions separated by an adhesive spacer portion having fluid channels therein within which a fluid to be assayed passes from an inlet port to a detection zone, wherein at least a portion of the adhesive spacer portion comprises a hydrophilic heat-sealable adhesive or a hydrophilic pressure-sensitive adhesive and is hydrophilic in character to increase the surface energy of the fluid flow path to enhance the flow of biological fluids in the channel. At least a portion of the surfaces of said base portions can be hydrophilic in character.

**Brief Description of the Drawings**

Figure 1 is a depiction of capillary rise in a cylinder;
Figure 2 is a depiction of the wetting of a fluid on a smooth flat surface;
Figure 3 graphically depicts the effect of surface treatment on contact angle;
Figure 4 depicts a laboratory coating technique for casting adhesive on a film;
Figure 5 depicts a method for contact angle measurement on a flat surface;
Figure 6 depicts a microfluidic device used in in-vitro sample analysis;
Figure 7 depicts the effect of surfactant concentration on contact angle;
Figure 8 depicts water contact angle vs. spreading time for hydrophilic films;
Figure 9 depicts water contact angle vs. surfactant concentration for films;
Figure 10 depicts the effect of surfactant concentration on contact angle and flow rate;

Figure 11 is a view in perspective of a microfluidic diagnostic device according to the present invention;

Figure 12 is a cross-sectional view of the device of Figure 11;

Figure 13 is a view in perspective of a reference example of a microfluidic device having an adhesive spacer portion attached to a base portion;

Figure 14 is a view in cross-section of the microfluidic device of Figure 13 wherein both base portions are present;

## Detailed Description of the Invention

Hydrophilic adhesives or films may be formulated to be thermally bonded or pressure sensitive. The hydrophilicity of the surface of the adhesive or film is controllable through the chemical structure, concentration and distribution of the surfactant in the adhesive coating. The hydrophilic properties reduce the surface tension of biological fluids (e.g., blood, urine, and sputum), thus allowing the rapid transfer of fluid from an inlet area to a remote reagent area in an in-vitro diagnostic device.

Two approaches can be used to improve the flow of biological fluids through a diagnostic device. One approach is to increase the surface energy of the substrate (or membrane) with various surface treatments. A second approach is to reduce the surface tension of the biological fluid.

Adhesives are typically hydrophobic polymers with a surface energy ranging from 30 to 40 dyne $cm^{-1}$ An approach to increase the flow properties of in-vitro diagnostic devices is to increase the surface energy of the hydrophobic adhesive coating. There are a number of patents that describe the synthesis and utility of hydrophilic polymers and adhesives.

For example, U.S. Patent No. 3,686,355 describes a block copolymer of a base polymer with a second surface modifying additive. U.S. Patent Nos. 5,354,815 and 5,614,598 describe polymers having enhanced hydrophilicity and thermal regulated properties. In this area, a hydrophilic polysiloxane anionic polymer is bonded to an aliphatic polyamide or polyester polymer fiber to enhance the hydrophilic and thermal properties of the textile. A number of U.S. and foreign patents are directed to the use of hydrophilic polymers used to formulate pressure sensitive adhesives. See, for example, U.S. Patent No. 5,508,313 (hydrophilic pendant moieties on polymer backbone), U.S. Patent No. 5,660,178 (hydrophilic crosslinking agents), U.S. Patent No. 6,121,508 (lipophilic pressure sensitive adhesive with a surfactant for skin contact in biomedical electrodes), WO 00/56828 (use of hydrophilic ester monomers that are polymerized to produce a wet stick pressure sensitive adhesive), EP 869979B (preparation of hydrophilic pressure sensitive adhesive using polar monomers), U.S. Patent No. 5,685,758 (hot melt adhesive with improved wicking for application to non-woven fabric), WO 97/48779 (hydrophilic hot melt adhesive composition prepared by blending adhesive components with a surfactant), and U.S. Patent No. 6,040,048 (water removable pressure sensitive adhesive containing hydrophilic pendent groups).

Polymeric films having modified surface properties are well known and produced by many distinct methods. See, for example, U.S. Patent Nos. 2,502,841 (gaseous chlorine); 2, 829, 070 (halogen gas); 3,142,582 (acid bath); 3,326,742 (halogenated organic amine); 3,561,995 (reactive conditioning agent with metal ion); 3,843,617 (aqueous acidic solution); 3,968,309 (surfactant-containing curable coating); 4,190,689 (titanium dioxide treatment); 4,387,183 (grafting hydrophilic chains to polymer surface); 4,416,749 (irradiation and surface hydrolysis); 4,460,652 (grafted hydrophilic polymer coating); 4,595,632 (hydroxy-fluorocarbon graft surface treatment); 4,666,452 (surface modified by hydrogen sulfato groups); 5,273,812 (hydrophilic film of hydrophilic monomer together with surface active agent); 5,280,084 (surface modification with carboxyl, carbonyl and hydroxyl groups followed by reaction with heterocyclic compound); 5,332,625 (crosslinked polymer surface); 5,451,460 (coating of non-ionic, hydrophilic surfactant in binder); 5,503,897 (irradiation and alkalization of polymer surface).

The present invention involves the use of adhesives in in-vitro diagnostic devices. Hydrophilic substrates or constructions can be hydrophilic heat seal coatings as well as pressure sensitive adhesive tapes. The combination of a pressure-sensitive or heat-sealable adhesive with hydrophilic properties to aid lateral flow and the wicking of biological fluids will prove beneficial to device manufacturers. Benefits will include increased flexibility in device design, increased wicking rates and consequently faster test results. Increased wicking consistency and potentially reduced sample volume are some of the advantages to be achieved through the use of hydrophilic pressure sensitive adhesives and heat-sealable coatings.

In view of the above, the objects of the present invention include providing adhesive coatings or films with controllable hydrophilicity to increase the surface energy of the fluid flow path to enhance the flow of biological fluids in microfluidic in-vitro diagnostic devices, providing hydrophilic adhesives that bond components of the diagnostic device thereby facilitating a more efficient manufacturing process for production of the device, increasing the transfer rate of the sample fluid from an inlet port to distal reagents and therefore reducing the time for analysis, enabling smaller sample volumes by enabling more efficient transport of fluid to a sensing reagent, and reducing risk of chemical interference by providing a wicking surface that allows an increased separation between the sampling port and the test reagents.

Hydrophilic coatings or films formulated by mixing surfactants with a polymer resin enhance the wicking of biological fluids into or through an in-vitro diagnostic medical device. Polymer resins may be selected from film forming polymers with a suitable glass transition temperature to form a hydrophilic coating. Similar resins may be selected for heat sealable hydrophilic coatings. In addition, resins typically used as pressure sensitive adhesives may be formulated with surfactants to provide a hydrophilic pressure sensitive adhesive. These constructions are dual functional in that they may serve to bond the components of the diagnostic device together and also to create high energy surfaces which reduce the surface tension of the biological fluid. The reduced surface tension of the fluid allows rapid transfer of the fluid from an inlet area to a remote reagent area in an in-vitro diagnostic device. The rapid fluid spreading can reduce the time needed for analysis. Since a smaller sample volume is required due to effective fluid wicking, device design flexibility is enhanced. This permits more efficient manufacturing processing with the potential for reduced product cost.

Hydrophilic adhesives can also be employed which do not require the incorporation of the surfactant into the formulation to provide the necessary hydrophilic properties. Examples of hydrophilic coatings and adhesives include polymers that can be cross-linked using di-hydroxyl terminated polyethylene glycol or polypropylene glycol monomers such as polyethylene glycol 600 supplied by Union Carbide Corporation. In addition, a vinyl terminated monomer with a hydrophilic moiety such as an anionic group can be grafted onto a polymer backing to increase the hydrophilic properties of the backing. One monomer that can be used is sodium AMPS, which is the sodium salt of 2-acrylamide-2-methyl-propanesulfonic acid, supplied by Lubrizol, which can be grafted onto the surface of a polymer by use of UV radiation. Such hydrophilic coatings can be used with and without the addition of a surfactant to provide a hydrophilic adhesive.

Surface tension of a fluid is the energy parallel to the surface that opposes extending the surface. Surface tension and surface energy are often used interchangeably. Surface energy is the energy required to wet a surface. To achieve optimum wicking, wetting and spreading, the surface tension of a fluid is decreased and is less than the surface energy of the surface to be wetted. The wicking movement of a biological fluid through the channels of a diagnostic device occurs via capillary flow. Capillary flow depends on cohesion forces between liquid molecules and forces of adhesion between liquid and walls of channel. The Young/Laplace Equation states that fluids will rise in a channel or column until the pressure differential between the weight of the fluid and the forces pushing it through channel are equal. Walter J. Moore, Physical Chemistry 3rd edition, Prentice-Hall, 1962, p. 730.

$$\Delta p = (2\gamma \cos\theta) / r$$

where $\Delta p$ is the pressure differential across the surface, $\gamma$ is the surface tension of the liquid, $\theta$ is the contact angle between the liquid and the walls of the channel and r is the radius of the cylinder. If the capillary rise is h and $\rho$ is the density of the liquid then the weight of the liquid in the column is $\pi r^2 gh\rho$ or the force per unit area balancing the pressure difference is $gh\rho$.

Therefore $(2\gamma\cos\theta)/r = gh\rho$ or $h = 2\gamma\cos\theta/g\rho$. For maximum flow through membranes (fluid wicking), the radius of the channel should be small, the contact angle $\theta$ should be small and $\gamma$ the surface tension of the fluid should be large.

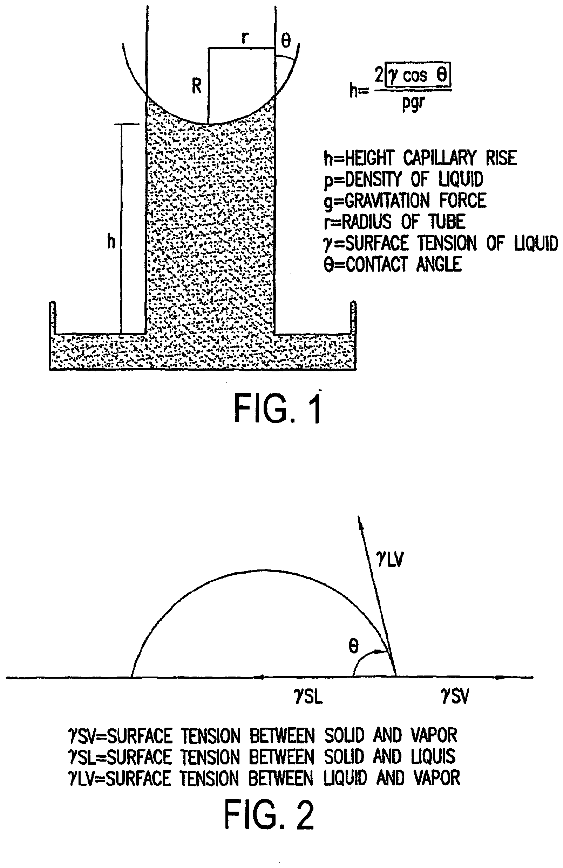

Wetting is the adhesion on contact between a liquid and solid. W.A. Zisman, "Influence of Constitution on Adhesion", Handbook of Adhesives, 2nd edition, Van Nostrand Reinhold Co., 1977, p. 38. For maximum wetting, the surface tension of the liquid must be less than or equal to the surface tension of the solid surface. This is the critical wetting tension of the solid. Figure 2 illustrates surface wetting of a fluid on a flat smooth surface.

The theoretical explanation of this phenomenon can be described by the classical model know as Young's Equation. T. Young, Philos. Trans. Roy. Soc. London, 95 (1805) p. 65.

$$\gamma_{SV} = \gamma_{SL} + \gamma_{LV} \cos\theta \text{--------------------Eq.1}$$

The diagram shown in Figure 2, illustrates the relationship between the contact angle $\theta$ and surface tension of liquid $\gamma_{LV}$ and solid $\gamma_{SV}$. W.A. Zisman, ibid, pp. 33-64. When the contact angle $\theta$ between liquid and solid is zero or so close to 0, the liquid will spread over the solid.

The spontaneous process of wettability can also be derived from the differential between work of adhesion and cohesion by substitution of Dupre Equation below in Equation 2:

$$W_A - W_C = \gamma_{SV} + \gamma_{LV} - \gamma_{SL} - 2\gamma_{LV} = \gamma_{SV} - (\gamma_{LV} + \gamma_{SL}) \text{-------------Eq.2}$$

This equation implies that spontaneous spreading will occur if the work required separating the liquid-solid interface is greater than liquid separation itself. Therefore, Equation 2 can be further derived by introducing the initial spreading coefficient S defined by Harkins ("The Physical Chemistry of Surface Films", Reinhold, 1952) and shown in Equation 3 below:

$$S = W_A - W_C = \gamma_{SV} - (\gamma_{LV} + \gamma_{SL})\text{------------------Eq.3}$$

Since $\gamma_{SL}$ is relatively small in comparison with $\gamma_{LV}$ , the initial spreading coefficient term becomes:

$$S = \gamma_{SV} - \gamma_{LV} \text{--------------------Eq.4}$$

Spreading is the movement of liquid across a solid surface. Contact angle is a measure of wettability. Spreading increases as the contact angle decreases until wetting is complete. Hence, the spreading will occur spontaneously when S is greater than zero, which also indicates that the surface tension of the solid must be greater than that of the liquid, as shown in Equation 4. From the initial spreading coefficient equation showed above (Eq. 4), the wettability will occur either by increasing surface tension of the solid or decreasing surface tension of liquid.

Surface treatments can be used to increase the surface energy of a solid include both physical and chemical methods. Corona discharge, mechanical abrasion, flame and plasma treatment are techniques used to increase surface energy. P.H. Winfield et al, "The Use of Flame Ionization Technology to Improve the Wettability and Adhesive Properties of Wood", Int'l Journal of Adhesion and Adhesives, Vol. 21(2), 2001. Chemical surface treatments include cleaning, priming, coating and etching to change the surface energy. Corona discharge treatment is the most widely used technique for surface treatment of plastics. During the treatment, the plastic surfaces are heavily bombarded with oxygen radicals at high-energy radiation levels. Consequently, the plastic surface either undergoes electret formation (J.M. Evans, J. Adhesion, 5(1973) pp. 1-7) or chemical structural changes (J.M. Evans, J. Adhesion, pp. 9-16; D.K. Owens, J. App. Polymer Science, 19(1975), pp. 275-271 and 3315-3326). Either proposal will improve the wettability of plastics. Another commonly used method is wet chemical treatment. This treatment involves oxidizing the plastic surface through exposure to oxidizing acids such as a mixture of chromic acid and sulfuric acid. (D. Briggs et al, Journal Material Science, 11 (976))

Six commonly used industrial plastics were selected for study. General information for each plastic is listed in Table 1:

Table 1

| General Information for Selected Plastics | | |
| --- | --- | --- |
| Plastic & Abbreviation | Product Name | Manufacturer |
| Polypropylene (PP) | Amoco™ Polypropylene | Amoco Chemical Company |
| High Density Polyethylene (HDPE) | Petrothene™ HD 5003C | Quantum Chemical Corporation |
| Polycarbonate (PC) | Cyrolon™ UVP PC | Cyro Industries |
| Polyethylene terephthalate (PET) | Rynite™ | Du Pont |
| Poly methyl methacrylate (PMMA) | Acrylite™ | Cyro Industries |
| Acrylonitrile-butadiene-styrene terpolymer (ABS) | CycolaC™ GPX3700-1000 | GE Plastics |

Each plastic was treated using two methods :1) corona discharge and 2) chromic acid. The corona discharge treatment involved exposing the surface of each plastic to an electric discharge of 10,000 to 50,000 volts at a frequency of approximately 500 kilohertz for approximately 5 seconds. The chromic acid treatment required the plastic surface to be flooded with chromic acid for 15 seconds then the acid was removed by washing with distilled water then rinsing the surface with isopropanol then wiped dry. The contact angle was measured immediately after drying or corona treatment using the method described below. The contact angle was measured on each plastic to quantitatively determine the effect of each treatment on the surface energy.

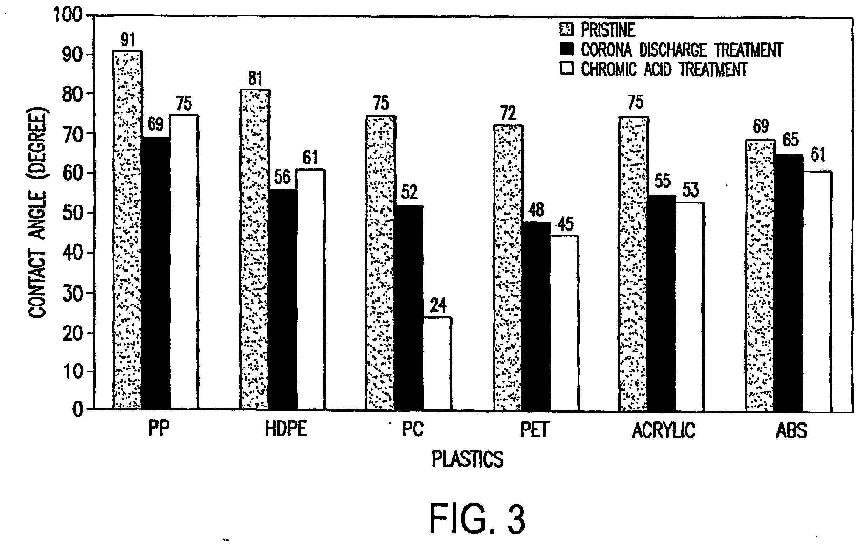

The data in Figure 3 shows that water contact angles on treated plastics decrease indicating an increase in surface energy. Consequently, the wettability of biological fluids will also be enhanced as a result of these treatments. Both corona discharge and chromic acid treatments were effective in improving the wettability of the surfaces. Corona discharge was most effective in increasing the surface energy of the polyolefin films (PP and HDPE) while chromic acid was more effective on plastics with more reactive groups such as polycarbonate and polyester panels. The corona discharge treatment method could improve the water contact angle by orienting surface electrical charges or by introducing oxygen on the surface. Either mechanism will increase the polarity of the plastic and thereby increase its surface tension. Consequently, the contact angle $\theta$ will be smaller due to reduced difference in surface tension between the plastic $\gamma_{SV}$ and the water $\gamma_{LV}$. A disadvantage of corona discharge treatment is the instability of the treatment. Corona treated substrates should be coated soon after treated.

The use of surfactants to lower the surface tension of a fluid is well known. M.J. Rosen, Surfactant and Interfacial Phenomena John Wiley & Sons, New York, (1978); Th.F. Tadros, Surfactants, Academic Press, Inc. New York, (1984); A.C. Clark et al, "New and Improved Waterborne Systems", Adhesives Age, September (1999), 33-40.

The effect of surfactants in coatings and adhesives has been studied to determine their effect on wettability, fluid flow rate and adhesive properties. Each surfactant was formulated into a base adhesive at different concentrations. The water contact angle was measured to determine the effect of surfactant on reducing the surface tension of the water.

### TABLE 2

| Physical Properties of Selected Surfactants | | | |
| --- | --- | --- | --- |
| Chemical Description | Structure | Charge Types | Mol. Wt. |
| Sodium 2-Ethylhexyl Sulfate | Branched | Anionic | 232 |
| Sodium Lauryl Sulfate | Linear | Anionic | 288 |
| Sodium Nonylphenol Ether Sulfate | Aromatic | Anionic | 498 |
| Nonylphenol Ethoxylate | Aromatic | Nonionic | 704 |
| Polyalkyeneoxide Modified | Linear | Nonionic | 600 |
| Heptamethyltrisiloxane | Siloxane | | |

Hydrophilic coatings and heat-sealing and pressure sensitive adhesives were prepared. Dissolution of polymeric resins occurred in organic solvents. Dissolution was followed by measurement of solution solids and viscosity over a period of several hours.

The surfactant was introduced into the liquid polymer mixture after dissolution of the resin. Gentle agitation for several minutes was sufficient to achieve homogeneity. Hydrophilic pressure-sensitive formulations were prepared by the introduction of a surfactant into liquid acrylic adhesive solutions and emulsions followed by gently mixing until dispersed or dissolved.

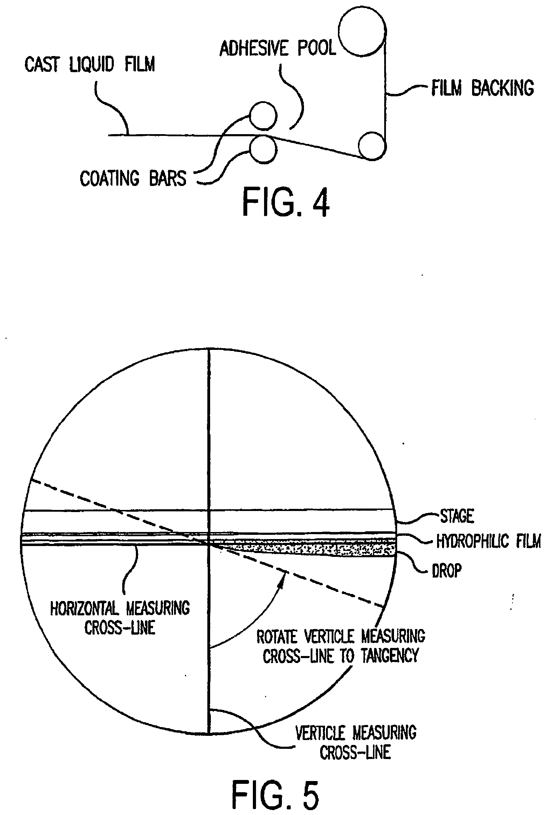

Hydrophilic films were prepared in the lab using coating apparatus. The lab preparations were accomplished by use of coating bars that evenly spread the liquid formulations on a film backing as shown in Figure 4. The liquid adhesive was first deposited as a pool onto a film backing, then the backing drawn through two stainless steel bars until the adhesive solution spread across and down the film to produce an even coating thickness. The thickness of the film was controlled the gap set between the two coating bars. The cast films were dried for five to ten minutes in a Blue M Stabil Therm convection oven set at 105°C. The dried coatings had an approximate thickness of 0.0005 to 0.001 inches (1.25 - 2.5 um), as measured with a Mitutoyo Absolute Digital Thickness Gauge. The hydrophilic adhesive coatings were protected with a film substrate of low surface energy (release liner).

The hydrophilic coatings were tested for surface wetting using de-ionized water. The sessile drop method was employed to measure the contact angle liquid water makes with the surface of the hydrophilic thin film. A ramé hart contact angle goniometer was used.

A micropipette was used to draw deionized water from a beaker. Several drops of the liquid were dispensed back into the beaker to ensure a bubble free liquid. The micropipette was then mounted onto the goniometer.

An approximate 1" " x 1 (2.5 x 2.5 cm) sample of hydrophilic film was place on the goniometer stage with the hydrophilic surface towards the drop. The film was flattened then secured to the stage by placing a magnet or clamps on each side of the film. Gloves were used when handling the film surface to avoid any oils or dirt from hands that could alter the surface of the film.

The micropipette was then lowered to just above the hydrophilic surface. A drop of water with a volume of approximately 2 $\mu$1 was suspended on the tip and lowered towards the film until the water drop dispensed onto the surface. The drop of water was allowed to spread across the surface until equilibrium was established (30 seconds). The microscope was focused to view the extreme left or right of the resulting drop (see Figure 5). The cross-line inside the scope was adjusted to tangency above the base of the drop to create a wedge of light bounded by the two cross-lines and the drop profile. The cross-line was slowly rotated while adjusting the cross travel of the specimen stage assembly so that the wedge of light is gradually extinguished and the cross-line attains tangency with the drop profile at the base of the drop. The contact angle was read directly from the scope reticle at the six o'clock position. The contact angle was recorded to the nearest degree on both sides of the spread water drop.

Samples of the hydrophilic pressure sensitive and hydrophilic heat seal adhesive substrates were tested for peel adhesion to stainless steel panels. Testing was performed on a MTS Alliance RT/1 mechanical tester equipped with a 25-lb (11.3 kg) load cell and hydraulic grips. The machine was interfaced with a Dell Optiplex GXlp computer system containing MTS TestWorks software package and Hewlett Packard 895c printer.

The hydrophilic pressure sensitive adhesive tapes were tested for peel strength from 6" x 6" (15.25 x 15.25 cm) stainless steel panels using Adhesives Research ART 1005, "Five Minute Peel". The method is similar to ASTM D3330-83. The testing was carried out in a controlled temperature (70° F; 21 °C) and humidity (50% RH) environment. Prior to testing, the stainless steel panels were cleaned with high purity urethane grade 2-butanone. The tape samples were cut to 1" x 10" (2.5 x 25 cm), then laminated (two passes) to the stainless steel panel using a 4.5-lb (2 kg), 80 durometer hardness roller. Peel testing was initiated after a five-minute dwell, by attachment of stainless steel plate to the bottom set of grips and overhanging, unbound portion of tape to the top set of grips. The tape was pulled away from the stainless steel plate at a rate of 12 inches (30 cm)/minute and at an angle of 180-degrees. The load and displacement were observed to increase to a maximum over the first 1-inch (2.5 cm) of the test then remain constant until the test was complete. The peel strength was calculated from the quotient of average load (oz) between one and five-inch (2.5 - 12.5 cm) displacement on the panel, and the sample width (in; cm).

Hydrophilic heat seal coatings were tested for adhesion using ASTM D1876-95. The testing was carried out at 70°F (21°C) and 50 % relative humidity. Adhesion was tested after lamination to a cleaned 7-mil (18 $\mu$m) polyester film. The samples were heat laminated on a Wabash press by exposure for 2 seconds at 100°C and at a pressure of 30-40 psi (200 - 275 kPa). The samples were aged four days at 70°F (21°C) and 50% R.H. Peel testing was conducted as described above and found to be acceptable.

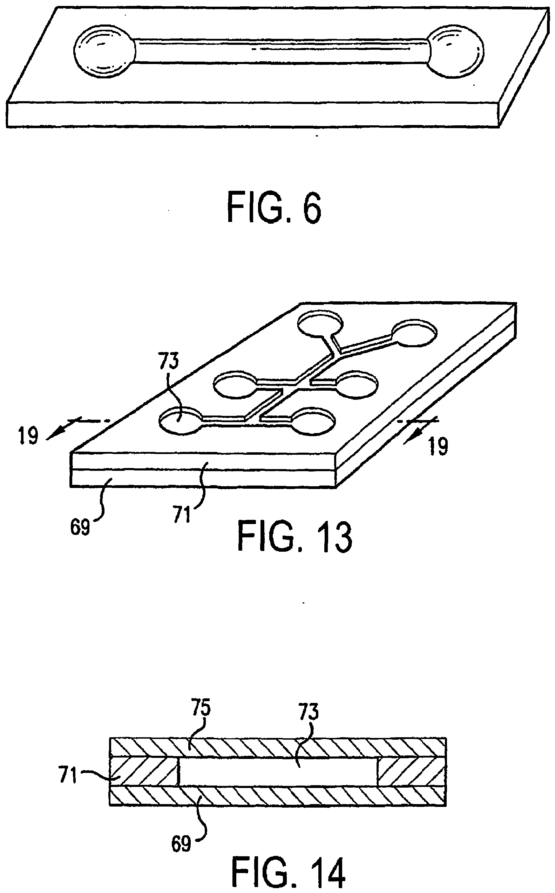

The effect of hydrophilic coatings and adhesives on the flow rate of distilled water in a microfluidic channel was investigated. Following a screening of the effect of different types of surfactants on contact angle, the most effective surfactants were formulated into adhesive tapes that were used as a cover for a microfluidic device as shown in Figure 6.

The microfluidic channel was molded in a device made from polystyrene. The channel had a length of 20 cm with a depth of 10 microns and a width of 30 microns. The hydrophilic tape was used to close the channel to create the microfluidic device. Distilled water was placed in one of the terminal wells and the time for the water to flow through the channel was measured.

Chemical surface analysis of the hydrophilic coatings was performed using infrared spectroscopy via attenuated total reflectance (ATR). The spectra were recorded using a Pike Miracle ATR single bounce sample port unit using a ZnSe crystal. Film samples were compressed onto the crystal using the compression arm at full contact pressure. Infrared spectra were collected using a MIDAC M 1300 Series FT-IR bench with a mercury-cadmium-telluride (MCT) nitrogen cooled detector. Absorbance spectra were collected from 30 scans per sample from 4000cm$^{-1}$ to 600cm$^{-1}$ at 2 cm$^{-1}$ resolution at a gain of 1X. The FTIR bench was interfaced with YKE Microsystem computer and analyzed using Grams 32 software package.

The surface topography of the hydrophilic coatings was observed using atomic force microscopy. The instrument used was a Digital Instruments Nanoscope IIIa Multimode instrument. Hydrophilic tapes were mounted onto 1-cm diameter magnetic stubs and imaged in the tapping mode. Using this mode, the AFM cantilever is oscillated at its resonant frequency. Contact between the oscillating tip and the tape surface causes a decrease in the measured amplitude of oscillation. Since the contact is made at the largest displacement from the cantilever equilibrium position, little energy is transferred to the sample and minimal deformation of the sample occurs. Images were obtained by raster scanning the sample surface under the tip and recording the z motion of the sample necessary to maintain constant amplitude during the scan. This mode of imaging has several advantages over direct contact mode imaging. Lateral forces that are prevalent during contact mode scans are eliminated. Additionally, this tapping mode provides a non-destructive method for the imaging of soft samples. Importantly, phase images obtained using the tapping mode can give additional information concerning the mechanical and adhesive properties of the sample surface. A. Doring et al, "Atomic Force Microscopy: Micro- and Nano-Mapping of Adhesion, Tack and Viscosity", 23rd Annual Technical Seminar: Pressure Sensitive Adhesive Tapes for the New Millennium, May, 2000, pp. 213-222.

All samples were initially scanned in air. The hydrophilic coating HY-10 was then rinsed with de-ionized water for 10 seconds before being wiped dry with a paper tissue. The sample was left to dry overnight and imaged the next morning.

Various surfactants as shown in Table 2 were formulated into an emulsion pressure sensitive adhesive.

**TABLE 2**

| Physical Properties of Selected Surfactants | | | |
|---|---|---|---|
| Chemical Description | Structure | Charge Types | Mol. Wt. |
| Sodium 2-Ethylhexyl Sulfate | Branched | Anionic | 232 |
| Sodium Lauryl Sulfate | Linear | Anionic | 288 |
| Sodium Nonylphenol Ether Sulfate | Aromatic | Anionic | 498 |
| Nonylphenol Ethoxylate | Aromatic | Nonionic | 704 |

(continued)

| Physical Properties of Selected Surfactants | | | |
|---|---|---|---|
| Chemical Description | Structure | Charge Types | Mol. Wt. |
| Polyalkyeneoxide Modified Heptamethyltrisiloxane | Linear Siloxane | Nonionic | 600 |

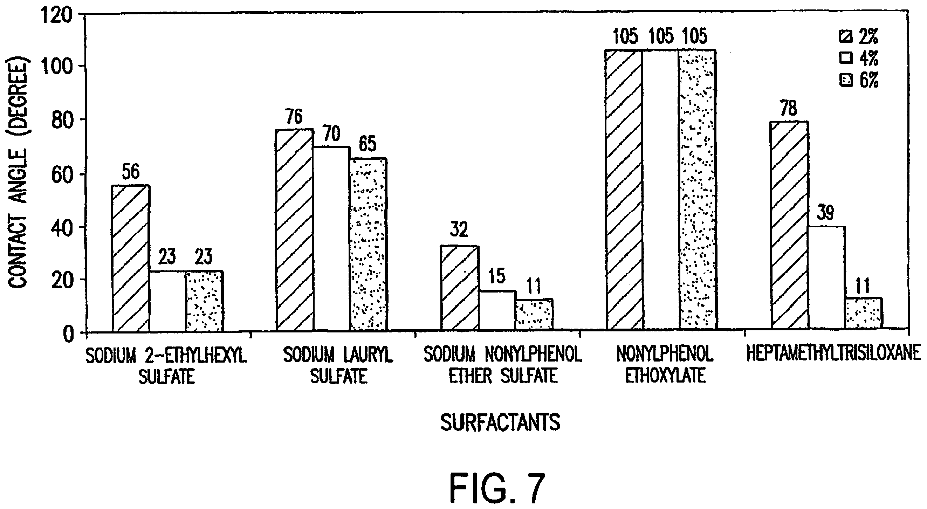

Figure 7 shows that most test samples exhibit a similar trend of decreasing contact angle with increasing surfactant concentration. Sodium nonylphenol ether sulfate exhibited the most effective surface tension reduction of water at all three surfactant concentrations used in this study. The nonionic surfactant, nonylphenol ethoxylate, exhibited little effect on the contact angle of de-ionized water. This may be due to its higher molecular weight and the lower water affinity of the hydrophilic group compared to anionic type surfactants. In addition, the nonylphenol group enhances its absorption onto the polymer surface. Polyalkyeneoxide modified heptamethyltrisiloxane (PMHS) (SILWET™ L77 from Union Carbide), also a non-ionic surfactant, reduced the water contact angle of the adhesive surface compared with the nonylphenol ethoxylate. PMHS has a siloxane polymer backbone instead of a hydrocarbon backbone, which accounts for its lower surface energy. In addition, PMHS also has a lower molecular weight than nonylphenol ethoxylate which enhances its mobility within the adhesive matrix. PMHS can be formulated into a solvent-based pressure sensitive adhesive in amounts of up to 20% by wt. to increase the hydrophilic properties of the adhesive.

Of the surfactants evaluated, sodium nonylphenol ether sulfate had the highest molecular weight of the anionic surfactant used. It is believed that the lower molecular weight anionic surfactants have better solubility into the adhesive matrix so that the concentration of the surfactant at the water/adhesive interface is less. The linear structure of sodium lauryl sulfate may improve its solubility into the adhesive so that its effect on the adhesive surface is less than that of sodium 2-ethylhexyl sulfate.

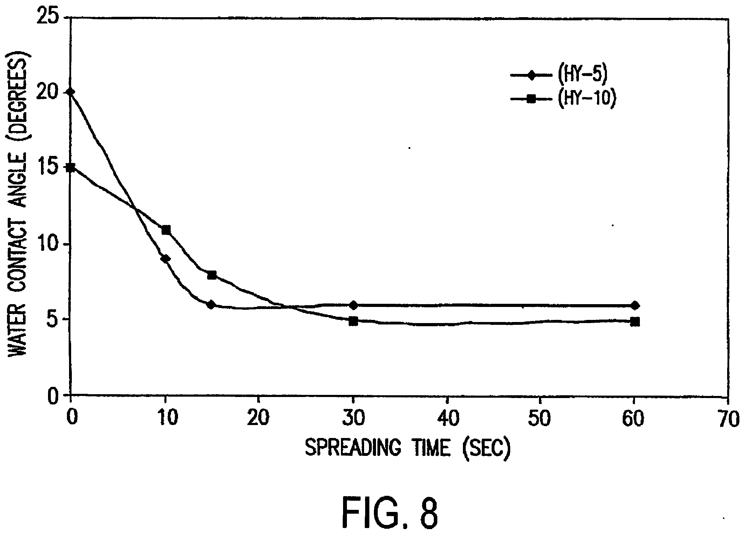

The wetting of the surface of HY-5 and HY-10 which are two hydrophilic heat seal adhesives was investigated by measuring the spreading of water. These hydrophilic heat seal adhesives were formulated using polyester resins and the anionic surfactants, sodium nonylphenol ether sulfate and sodium dioctylsulfosuccinate, respectively. Figure 8 is a graph that describes the spreading behavior of water on the surface of HY-5 and HY-10 thin film coatings. Water was dropped onto the surface of the adhesives and the contact angle was measured as a function of time. Initially there is rapid spreading of the drop as it contacts the surface of the film. The contact angle decreases quickly to less than 10 degrees. Equilibrium is established within thirty seconds to one minute. This spreading behavior is typical of the hydrophilic coatings, heat-seal adhesives, and pressure sensitive adhesives.

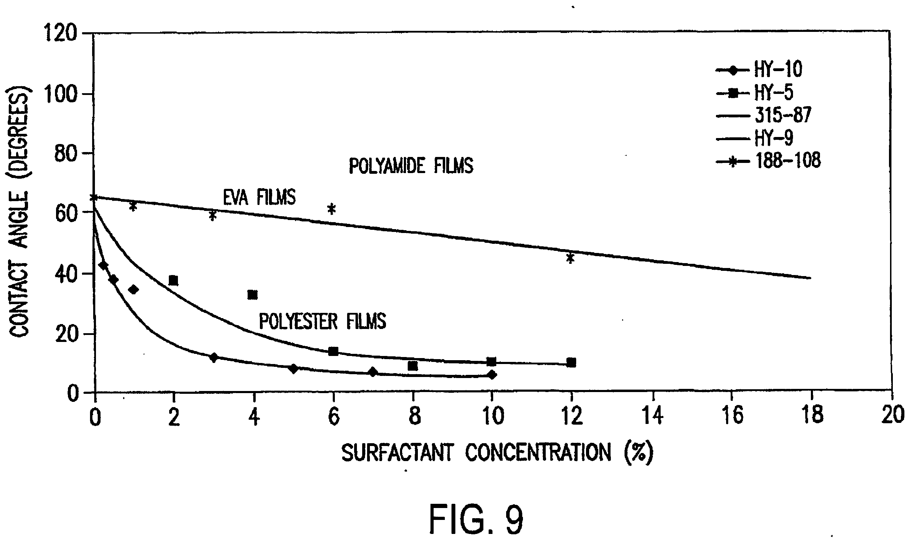

Figure 9 shows the effect of the surfactant concentration on the surface wettability of the dried films prepared using different polymeric resins. Polyamide, ethylene vinyl acetate, and polyester resins were formulated with sodium dioctylsulfosuccinate. The resins studied included films of polyamide, ethylene vinyl acetate, and polyester chemistries. When no surfactant is present in the coatings the contact angle is high since the polymeric resins are hydrophobic. By increasing the surfactant concentration the surface becomes more hydrophilic and lower water contact angles are observed indicating significant surface wetting. At very high surfactant concentrations the wetting effect can be enhanced or attenuated depending on the surfactant and its compatibility with the polymer matrix.

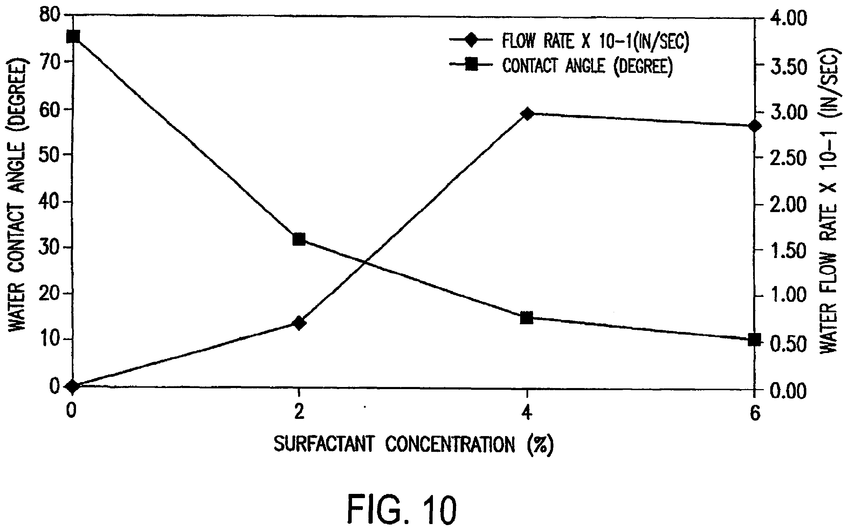

Figure 10 shows the effect of surfactant concentration on the rate of water flow in a covered microfluidic device (corresponding to device of Fig. 6). In this experiment, a hydrophilic pressure sensitive adhesive was formulated using concentrations of sodium nonylphenol ether sulfate ranging from 0 to 6 percent. When there was no surfactant added to the adhesive, water did not flow through the channel. With increasing concentration of surfactant the rate of water flow through the microchannels increased while the contact angle decreased.

The increased flow rate of water can be attributed to the reduction of water surface tension. The principle that could be used to explain this phenomenon is capillary rise. The height of the water in the capillary is determined by a factor of two times the product of liquid surface $\gamma_{LV}$ and coso regardless of liquid density and gravitation force. As a result, the water will advance further when the surface tension of water is close to the surface tension of the capillary material that is now determined by the hydrophilic adhesive cover. At high surfactant concentration (greater than 4% in Figure 10), the rate of flow levels off since the concentration of surfactant exceeds the critical micelle concentration. Additional surfactant on the surface of the adhesive does not reduce the surface tension of the fluid and may become autophobic. W.A. Zisman, "Influence of Constitution on Adhesion", Handbook of Adhesives, 2nd edition, 1977, p. 46.

Atomic force microscopy (AFM) was used to visualize the topography of hydrophilic coatings. The AFM images of coatings containing 0%, 1%, 5% and 10% surfactant were obtained. The images show enrichment of the film surface at the film/air interface with increasing amount of surfactant introduced to the adhesive formula. The AFM image of the coating containing no surfactant shows a relatively smooth, flat surface.

Transformation is observed when 1% or less surfactant has been incorporated into the adhesive coating, where raised surface features are observed on the film surface. Increased surface topography is observed at 5% surfactant while at 10% surfactant the surface appears to be smoother due to saturation of the surface.

Infrared spectra of the coatings confirm the increase in surfactant concentration on the surface. The prominent peak at 2958 cm$^{-1}$ in the ATR is assigned to the C-H stretch of a $CH_3$, group on the surfactant in the hydrophilic adhesive and is used to monitor surfactant accumulation on the surface. A plot of absorbance of the C-H stretch as a function of concentration of surfactant at 0%, 1.0%, 5.0% and 10% shows a flattening resulting from the surface saturation by the surfactant

## Example 1

(Hydrophilic Coating)

A polyester resin with a high glass transition temperature commercially available as Vitel™ 2200 BA from Bostik Chemical Company is dissolved in a solvent of methyl ethyl ketone and toluene (7:3 weight ratio). A commercial surfactant such as Rhodapex™ CO-436 available from Rhodia Inc. is dissolved in the resin solution to provide a surfactant solids to resin ratio of between 3:97 to 6:94. A hydrophilic coating is formed by spreading the resin/surfactant solution onto a polymer film and allowing the solvent to evaporate. Wetting the dried film surface with distilled water causes spreading of water on the surface. The contact angle of the water on the surface ranges from 5 to 10 degrees.

## Example 2

(Hydrophilic Heat Sealable Coating)

A similar formulation and coating as in Example 1 is prepared using a polyester resin with a lower glass transition temperature such as Vitel™ 3200 through 3500 series resins with a glass transition temperature between -15 °C to +15°C. One example is Vitel™ 3300B which has a Tg of+11°C. A heat sealable hydrophilic coating is formed by coating the formulation of resin and surfactant onto a surface such as a polymeric film and allowing the solvent to evaporate. The contact angle of the hydrophilic coating is similar to those of Example 1 (5 to 10 degrees). Other resins such as ethylene vinyl acetate and polyamide polymers may be used as heat sealable formulations.

## Examples 3-8

(Hydrophilic Pressure Sensitive Coatings - Aqueous Based)

A hydrophilic pressure sensitive coating is prepared by formulating an emulsion based resin such as Aroset™ 3500 available from Ashland Specialty Chemical Company (division of Ashland, Inc.), with a surfactant such as Rhodapex™ CO-433 available from Rhodia, Inc. The formulation was coated onto a hydrophobic polymer film such as 5 mil (12.5 μm) polyester film available from DuPont Teijin Films. After coating and drying the formulation, the contact angle was measured. The following table illustrates the effect of surfactant concentration on the contact angle which is related to the surface energy of the hydrophilic adhesive. The adhesive 180° peel force can be modified through addition of additives such as tackifiers.

| Acrylic Resin (%) | Rhodapex™ CO-433 | Contact Angle | 180° Peel |
|---|---|---|---|
| Aqueous Solvent | (%) | (Degrees) | (oz/inch) (g/cm) |
| 99 | 1 | 95 | |
| 98 | 2 | 32 | 68 (760) |
| 97 | 3 | 29 | |
| 96 | 4 | 15 | 6 (67) |
| 95 | 5 | 13 | |
| 94 | 6 | 11 | 7 (78) |

## Examples 9-12

(Hydrophilic Pressure Sensitive Coatings - Solvent Based)

Similar to Examples 3-8, hydrophilic pressure sensitive adhesive coatings are formulated using solvent based adhesives and surfactants. An acrylic resin adhesive in the organic solvent ethyl acetate was formulated with various concentrations of Rhodapex™ CO-433. After coating onto a polyester film and drying, the contact angle of the coating was measured. The following table shows the effect of surfactant concentration on the contact angle and 180° peel force.

| Acrylic Resin (%) Ethyl Acetate Solvent | Rhodapex™ CO-433 (%) | Contact Angle (Degrees) |
|---|---|---|
| 97 | 3 | 33 |
| 94 | 6 | 17 |
| 91 | 9 | 16 |
| 88 | 12 | 15 |

In addition to the concentration of the surfactant, the surface energy of the hydrophilic coating can be controlled by the selection of surface active agent. The selection of surface active agent is based on factors such as molecular weight, linear vs. branched structure, ionic vs. non-ionic and the type of ionic moiety present, aromatic vs. aliphatic structure. These chemical structure properties can be used to control the hydrophilic characteristics and surface energy of the coating. The following table shows the effect on contact angle of 2 percent surfactant in Aroset™ 3500 coatings by the selection of surfactant structure. The following table shows the effect of surfactant characteristics on coating wettability:

| Surfactant/Contact Angle | Ionic Charge | Molecular Wt | Structure |
|---|---|---|---|
| Sodium 2-Ethylhexyl sulfate/41° | anionic | 232 | branched |
| Sodium octyl Sulfate/19° | anionic | 232 | linear |
| Sodium lauryl Sulfate/20° | anionic | 288 | linear |
| Sodium Nonylphenol sulfate/32° | anionic | 382 | aromatic |
| Nonylphenol Ethoxylate/105° | nonionic | 820 | aromatic |

A novel feature of using a hydrophilic coating formulated with Rhodapex™ CO-436 (the ammonium salt of sulfated nonylphenol ethoxylate) is the ability to pattern the surface energy of a uniform coating using radiant energy. When thermal energy is applied to the coating in a pattern such as stripes, circles or any other configuration, the surface energy in the area of applied energy is reduced. It is believed that ammonia gas is evolved due to the thermal energy leaving the sulfonic acid of nonylphenol ethoxylate remaining. The hydrophilicity of the coating decreases and consequently becomes water resistant. Radiant energy sources such as lasers and electron beam may also be employed to cause the evolution of a labile cation to customize the physical character of the coating. This may be used with advantage in the production of in-vitro diagnostic devices, such as by the application of thermal energy to the surface to produce a parallel, laterally-oriented, striped pattern of alternating hydrophilic/hydrophobic areas. The presence of the hydrophobic areas may be employed with advantage to slow the wicking of the material to be tested as it travels from a hydrophilic region to a hydrophobic region, whereby additional time for reaction between the analyte and the reagent results. Fluid wicking through the device may be retarded over areas of lower surface energy to permit time for reaction or complex formation by use of a single coating. This may be employed to avoid too rapid fluid wicking which may be detrimental if the reaction time is insufficient. Of course, a series of reaction zones of various shapes and configurations can be created on a single film.

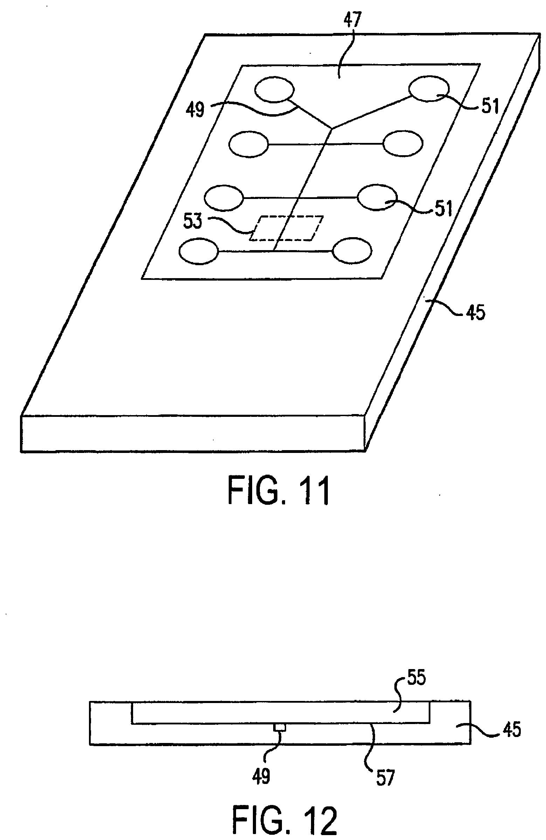

In the context of a microfluidic diagnostic device which employs capillary transport of the fluid sample during the analysis procedure, such devices typically include microfluidic channels molded in a suitable polymeric substrate (see Figures 6 and 11). Microfluidic devices generally refers to a device having one or more fluid channels (including passages, chambers or conduits) which have at least one internal cross-sectional dimension (width or depth) of between 0.1 pm and 500 mm within which a fluid sample passes from an inlet port to a detection zone.

The microfluidic diagnostic device is generally comprised of a substantially planar base portion having one or more microfluidic channels (including passages, chambers or conduits) therein. A variety of materials may comprise the base portion, including polymeric materials such as polymethylmethacrylate, polycarbonate, polytetrafluoroethylene, polyvinylchloride, polydimethylsiloxane, polysulfone, and silica-based substrates such as glass, quartz, silicon and polysilicon, as well as other conventionally-employed substrate materials.

Such substrates are manufactured by conventional means, such as by injection molding, embossing or stamping. The microfluidic channels may be fabricated into the base portion by conventional micro fabrication techniques known to those skilled in the art, including but not limited to photolithography, wet chemical etching, laser ablation, air abrasion techniques, injection molding, embossing, and other techniques. The base material is selected on the basis of compatibility with the desired method of manufacture as well as for compatibility with the anticipated exposure to materials and conditions, including extremes of pH, temperature, salt concentration, and the application of electric fields. The base material may also be selected for optional properties including clarity and spectral characteristics.

An enclosure surface or cover is placed over the top portion of the base substrate to enclose and otherwise seal the microfluidic channels. In the context of the present invention, the channels are covered with a substrate according to the present invention the surface of which is hydrophilic which covers the channels in the base substrate. The fact that the surface of the covering substrate is hydrophilic in nature enhances the flow of the liquid through the microfluidic channels. As discussed above, the hydrophilic covering substrate can comprise a variety hydrophilic pressure sensitive adhesive layer or a hydrophilic heat-sealable layer. Hydrophilic pressure sensitive adhesives can be bonded to the upper portion of the base substrate in covering/sealing relation to the microfluidic channels by application of pressure. Hydrophilic heat-sealable layers can be bonded to the upper portion of the base substrate in covering/sealing relation to the microfluidic channels by application of pressure and heat, with the temperatures employed being sufficient to cause bonding of the covering layer without adversely affecting the physical structure of the base material.

Such devices typically include optical detector means positioned adjacent to a detector window whereby the detector senses the presence or absence of an optical characteristic from within the microfluidic passage or channel resulting from flow of the liquid sample through the passage or sample. The optical detector may comprise any of a variety of detector means such as fluorescent, colorimetric or video detection systems, which include an excitation light source (laser or LED). A variety of optically detectable labels can be employed to provide an optically detectable characteristic such as colored labels, colloid labels, fluorescent labels, spectral characteristics and chemiluminescent labels.

As discussed above, an alternative to otherwise having to ensure that the channels possess sufficient hydrophilicity to cause the fluid sample to travel along the capillary tube, the top portion of the channel is covered with a hydrophilic material in accordance with the present invention. That is, a heat-sealable polymeric film having hydrophilic surface characteristics may be applied over the open cavity of the channel to both enclose the channel and provide the necessary hydrophilic character so that the fluid sample will be caused to wet the channel. As an alternative, the polymeric film may include a pressure sensitive adhesive coating which is also hydrophilic in character to provide the necessary hydrophilicity to cause the fluid sample to wet the channel. The use of such materials in the construction of the microfluidic diagnostic device also serves to simplify the manufacturing of the device. In the context of the present invention, the entire facing surface of the covering layer need not be hydrophilic; instead, only that portion of the covering layer that serves to enclose the microfluidic channels is required to be hydrophilic. Of course, certain portions of the covering layer that enclose the microfluidic channels may be rendered less hydrophilic than other portions to modify the flow rate of the fluid sample.

A typical microfluidic device which has been prepared in accordance with the present invention is depicted at Figures 11 and 12. The device of Figure 11 includes base portion 45, recess 47 in the top of the base 45, open microfluidic channels 49, fluid reservoirs 51 and viewing window 53. In the device of Figure 11, the microfluidic channels 49 are uncovered in order to depict the interior of the device. In the cross-sectional view of the device of Figure 11 (at Figure 12), base portion 45 includes microfluidic channel 49 which is shown to be enclosed by cover portion 55. Cover portion 55 includes a facing hydrophilic surface 57 whereby the fluid sample which enters the microfluidic channel 49 will contact the facing hydrophilic surface and cause the sample to be transported along the length of the channel. The facing surface 57 of the cover 55 is rendered hydrophilic by the presence of a hydrophilic pressure sensitive adhesive or a means heat-sealable adhesive.

A reference example of a microfluidic in-vitro diagnostic device is depicted in Figures 13 and 14. The microfluidic in-vitro diagnostic device may be comprised of opposing base layers 69, 75 separated by an adhesive spacer layer 71. While only a single base layer is shown in Figure 13 so as to depict the fluid channels 73, both base layers are shown in Figure 14. The spacer layer 71 may have fluid channels 73 provided therein within which a fluid to be assayed passes from a reservoir to a collection point. At least a portion of the surfaces of the base layers 69, 75 and the spacer layer which define the boundaries of the fluid channels are hydrophilic in character. The spacer layer 71 is an adhesive layer which is bonded to the opposing base layers, either as a result of pressure sensitive adhesive properties of the spacer layer or as a result of being heat-sealed to each of the base layers. If pressure sensitive, the spacer layer may be used in the form of a transfer film or as a double face construction. As discussed above, if the base layers are not hydrophilic in character, the spacer layer would possess the requisite hydrophilic character to assist wetting of the fluid channel by the fluid sample. The fluid channels 73 in the spacer layer may be die-cut into the spacer layer or provided by any other means effective to provide a spacer layer with the requisite fluid channels. One advantage of such a construction is that the micro-fluidic device may be constructed easily without the need to mold the fluid channels into the base layers as in the embodiment of Figure 11.

It is frequently a disadvantage in any determination by means of fluorescent detection that "background" fluorescence occurs which may affect the accuracy of the desired fluorescent detection. It has previously been proposed to employ "low background" assay platforms and well plates for use in fluorescent detection methods to minimize the degree of background fluorescence during the assay. See U.S. Patent Nos. 5,910,287 and 6,171,780 in this regard. These patents teach the use of polymers having low fluorescence and high transmittance such as cycloolefins in the formation of the bottom portion of the wells in a multi-well assay platform.

It would be desirable, however, to also employ a low fluorescent sealing or cover layer either alone or in conjunction with a low fluorescent assay platform or multi-well plates to further reduce the possibility of undesirable background fluorescence during the assay by fluorescent detection.

Fluorescence is defined as "radiative transition from the lowest excited singlet state $(S_1)$ to the ground state (So) (Electronic Properties of Polymers, ed. J. Mort et al, p. 177, 1982). Materials for applications where no or minimal fluorescence is preferred (such as microfluidic devices) typically have high excitation energy potential. In polymeric materials, the base monomer preferably has a high ionization potential and low electron affinity. High energy is required to excite the molecules from a ground state to an excited state. Low fluorescent compounds do not easily accept charge transfer from other compounds or excited states. Similarly, molecules that are easily polarized by the delocalization of an electron should be avoided. Aromatic compounds and compounds with a conjugated pi electron structure may be easily excited by a radiate excitation source due to their non-localized electrons.

Advantageously, such sealing or cover layers will exhibit low natural fluorescence at the excitation and detection wavelength used to detect the biomaterial; will be dimensionally stable and not flow into any microfluidic channels present; will adhere to the base plate without creating voids or gaps that may allow migration of the components from one channel to an adjacent channel; is compatible with the chemical reagents used in the microchannels and reservoirs such as electrophoretic media and biomaterials including DNA fragments and polypeptides; be compatible with the separation conditions employed including pH (e.g., pH of 2-12, preferably 3-8), electric field potentials and voltage gradients of 200 volts/cm; exhibit good stability to moisture and temperature change; preferably contain no charged substituents which may interfere with the separation of biomaterials that contain charged groups; contain no leachable components that may contaminate the sample; and exhibit little or no spectral emission in the wavelength range of 400 to 800 nM. Such sealing or cover layers may possess pressure sensitive adhesive properties or be heat sealable.

With respect to the material used in the assay platform, such material may be either flexible or rigid, but is preferable that such materials be clear and colorless; chemically compatible with electrophoretic separation; exhibit little or no fluorescence under assay detection conditions as evidenced by little or no spectral emissions in the wavelength of 400 to 800 nM; be dimensionally stable and withstand pressure during electrophoresis; and dissipate heat during electrophoresis; and have minimal cross-sectional dimension.

When the sealing layer comprises a pressure sensitive adhesive, it is preferable to use a liner to protect the sealing surface. If a liner is used, it is desirable that there is no transfer of compounds from the liner to the sealing surface which will interfere with the separation of biomaterials or increase the fluorescence of the sealing surface.

Materials suitable for use in the present invention which exhibit minimal or low fluorescence and which may be used as substrate materials include but are not limited to polyolefins, polysiloxanes, polyalkylmethacrylates, and polycarbonates. Examples of suitable substrate films include Rohm PLEXIGLASS™ S30K, Rohm OROGLASS™ , and Goodfellow Polymethylmethacrylate and Mitsubishi SKINKOLITE™ HBS 007.

Materials suitable for use in the present invention as sealing or cover materials which exhibit minimal or low fluorescence include but are not limited to the above materials as well as adhesives such as alkyl(meth)acrylic acid esters. Preferred adhesive compositions contain no aromatic moieties such as those found in aromatic solvents, aromatic monomers, polymerization inhibitors or polymerization initiators as the presence of the aromatic moiety will result in undesirable spectral emissions. If any aromatic solvents such as toluene or xylene are used in the formation of the adhesive, they are removed during the drying process or by subsequent treatment of the product. Silicone-based adhesives such as Dow™ 7657 or Sylgard™ 184 (polydimethyl siloxane) may also be used. A low fluorescence sealing layer may be provided with advantage comprised of amorphous polyolefins such as polyethylene, polypropylene or blends of polyolefins.

Preferred acrylate-based pressure sensitive adhesives are formulated using alkyl (alkyl)acrylate esters that are polymerized using non-aromatic initiator and cross-linkers. The concentration of unreacted components such a monomers, initiators, and crosslinkers should be minimized to ensure low fluorescence. Typically, the concentration of unreacted monomers in the formulation will be in the ppm range. When an organic solvent is used in the formulation, non-aromatic solvents such as low molecular weight hydrocarbons, alcohols, and esters are preferred. Solvents such as heptane, hexane, ethyl acetate and isopropanol are preferred. Reactive monomers such as acrylic acid may be inhibited using substituted hydroquinones. Substituted hydroquinones extend the shelf life of the reactive monomers and higher concentrations are used for the most reactive monomers. Substituted hydroquinones in the sealing layer may fluoresce significantly after exposure to radiant energy due to their low activation energy.

**Claims**

**1.** A method of manufacturing a microfluidic in-vitro diagnostic device, comprising:

providing a base having at least one fluid channel within which a fluid sample to be assayed passes from an inlet port to a detection zone; and

enclosing said at least one fluid channel with at least one enclosure surface,

wherein said at least one enclosure surface of the fluid channel comprises a hydrophilic heat-sealable adhesive or a hydrophilic pressure-sensitive adhesive and is hydrophilic in character to increase the surface energy of the fluid flow path to enhance the flow of biological fluids in the channel.

2. A method of claim 1, wherein said at least one enclosure surface is heat-sealed to said base to seal said at least one fluid channel by the hydrophilic heat sealable adhesive.

3. A method of claim 1, wherein the surface of said at least one enclosure surface facing said at least one fluid channel comprises a pressure sensitive adhesive.

4. A method of claim 1, wherein said enclosure surface is comprised of a low fluorescent material.

5. A method of claim 1, wherein said enclosure surface is comprised of a polymeric material with which a surfactant is admixed to provide hydrophilic surface properties.

6. A method of claim 1, wherein said enclosure surface is comprised of a polymeric material having a surfactant applied to a surface thereof.

7. A method of claim 5 or 6, wherein said surfactant is a non-ionic or anionic surfactant.

8. A method of claim 7, wherein said surfactant is selected from the group consisting of polyethylene oxide, polypropylene oxide, nonylphenol ethoxylate and polyalkyleneoxide modified heptamethyltrisiloxane.

9. A method of claim 7, wherein said surfactant is selected from the group consisting of sodium or ammonium salts of nonyl phenol ethoxyl sulfonic acid, sodium lauryl sulfate, sodium 2-ethylhexyl sulfate and sodium dioctylsulfo succinate.

10. A method of claim 7, wherein said surfactant is selected from the group consisting of the ionic salt of 2-acrylamido-2-methyl propanesulfonic acid, N-vinyl caprolactam, caprolactone acrylate, N-vinyl pyrrolidone, and sulfate and acrylic monomers.

11. A method of claim 1, wherein portions of the enclosure surface are less hydrophilic than other portions whereby the flow rate of a biological fluid flowing through the channel is modified.

**Patentansprüche**

1. Verfahren zur Herstellung einer mikrofluidischen In-vitro-Diagnosevorrichtung, umfassend:

Bereitstellen eines Trägers mit mindestens einem Fluidkanal, in dem eine zu untersuchende Test-Flüssigkeit von einer Einlass-Öffnung zu einer Untersuchungsbereich strömt; und

Verschließen des mindestens einen Fluidkanals unter Verwendung mindestens einer Umgebungsoberfläche, wobei die mindestens eine Umgebungsoberfläche des Fluidkanals einen hydrophilen Heißsiegelklebstoff oder einen hydrophilen drucksensitiven Klebstoff umfasst und hydrophilen Eigenschaften aufweist, um die Oberflächenenergie des Fluidströmungswegs zu erhöhen, um den Fluss biologischer Fluide im Kanal zu verbessern.

2. Verfahren nach Anspruch 1, wobei die mindestens eine Umgebungsoberfläche mit dem Träger heißgesiegelt wird, um den mindestens einen Fluidkanal unter Verwendung des hydrophilen Heißsiegelklebstoffs abzudichten.

3. Verfahren nach Anspruch 1 oder 2, wobei die Oberfläche der mindestens einen Umgebungsoberfläche, die dem mindestens einen Fluidkanal zugewandt ist, einen drucksensitiven Klebstoff umfasst.

4. Verfahren nach Anspruch 1, wobei die Umgebungsoberfläche aus einem niedrig fluoreszierenden Material besteht.

**5.** Verfahren nach Anspruch 1, wobei die Umgebungsoberfläche aus einem polymeren Material besteht, auf dessen Oberfläche ein oberflächenaktiver Stoff beigemischt wird, um hydrophile Oberflächeneigenschaften bereitzustellen.

**6.** Verfahren nach Anspruch 1, wobei die Umgebungsfläche aus einem polymeren Material besteht, auf dessen Oberfläche ein oberflächenaktiver Stoff aufgebracht ist.

**7.** Verfahren nach Anspruch 5 oder 6, wobei der oberflächenaktive Stoff ein nicht-ionischer oder anionischer oberflächenaktiver Stoff ist.

**8.** Verfahren nach Anspruch 7, wobei der oberflächenaktive Stoff aus der Gruppe bestehend aus Polyethylenoxid, Polypropylenoxid, Nonylphenolethoxylat und Polyalkylenoxid modifiziertes Heptamethyltrisiloxan ausgewählt ist.

**9.** Verfahren nach Anspruch 7, wobei der oberflächenaktive Stoff aus der Gruppe bestehend aus Natrium- oder Ammoniumsalzen von Nonylphenolethoxysulfonsäure, Natriumlaurylsulfat, Natrium-2-ethylhexylsulfat und Natriumdioctylsulfosuccinat ausgewählt ist.

**10.** Verfahren nach Anspruch 7, wobei ausgewählt ist aus der Gruppe bestehend aus dem ionischen Salz von 2-Acrylamido-2-methylpropansulfonsäure, N-Vinylcaprolactam, Caprolactonacrylat, N-Vinylpyrrolidon und Sulfat- sowie Acrylmonomere ausgewählt ist.

**11.** Verfahren nach Anspruch 1, wobei Teile der Umgebungsoberfläche weniger hydrophil sind als andere Teile, wodurch die Durchflussrate eines durch den Kanal fließenden biologischen Fluides modifiziert wird.

**Revendications**

**1.** Procédé de fabrication d'un dispositif de diagnostic in vitro micro-fluidique, ledit procédé comprenant les étapes suivantes :

fournir une base comportant au moins un tunnel de fluide à l'intérieur duquel un échantillon de fluide à analyser passe d'une ouverture d'entrée à une zone de détection ; et
fermer ledit au moins un tunnel de fluide à l'aide d'au moins une surface de fermeture, ladite au moins une surface de fermeture du tunnel de fluide comprenant un adhésif thermocollant hydrophile ou un adhésif hydrophile sensible à la pression et étant de caractère hydrophile afin d'augmenter l'énergie de surface du chemin d'écoulement de fluide afin d'améliorer l'écoulement de fluides biologiques dans le tunnel.

**2.** Procédé selon la revendication 1, ladite au moins une surface de fermeture est thermocollée à ladite base afin de sceller ledit au moins un tunnel de fluide à l'aide l'adhésif thermocollant hydrophile.

**3.** Procédé selon la revendication 1, la surface de ladite au moins une surface de fermeture qui fait face audit au moins un tunnel de fluide comprenant un adhésif sensible à la pression.

**4.** Procédé selon la revendication 1, ladite surface de fermeture est en un matériau faiblement fluorescent.

**5.** Procédé selon la revendication 1, ladite surface de fermeture étant en un matériau polymère sur lequel la surface auquel est mélangé un tensioactif afin de conférer des propriétés de surface hydrophiles.

**6.** Procédé selon la revendication 1, ladite surface de fermeture étant en un matériau polymère sur lequel un tela surface duquel est appliqué un tensioactif.

**7.** Procédé selon la revendication 5 ou 6, ledit tensioactif étant un tensioactif non ionique ou anionique.

**8.** Procédé selon la revendication 7, ledit tensioactif étant choisi dans le groupe comprenant l'oxyde de polyéthylène, l'oxyde de polypropylène, l'éthoxylate denonylphénol et le trisiloxane d'heptaméthyle modifié par un oxyde de polyalkylène.

**9.** Procédé selon la revendication 7, ledit tensioactif étant choisi dans le groupe comprenant les sels de sodium ou d'ammonium de l'acide nonylphénol-éthoxylsulfonique, le laurylsulfate de sodium, le 2-éthylhexylsulfate de sodium et le dioctylsulfosuccinate de sodium.

10. Procédé selon la revendication 7, ledit tensioactif étant choisi dans le groupe comprenant un sel ionique de l'acide 2-acrylamido-2-méthylpropanesulfonique, le N-vinylcaprolactame, l'acrylate de caprolactone, la N-vinylpyrrolidone et des monomères sulfate et acryliques.

11. Procédé selon la revendication 1, des parties de la surface de fermeture étant moins hydrophiles que d'autres parties, ce qui modifie le débit d'un fluide biologique circulant à travers le tunnel.

$$h = \frac{2\gamma \cos \theta}{pgr}$$

h=HEIGHT CAPILLARY RISE
p=DENSITY OF LIQUID
g=GRAVITATION FORCE
r=RADIUS OF TUBE
$\gamma$=SURFACE TENSION OF LIQUID
$\theta$=CONTACT ANGLE

FIG. 1

$\gamma$LV

$\theta$

$\gamma$SL          $\gamma$SV

$\gamma$SV=SURFACE TENSION BETWEEN SOLID AND VAPOR
$\gamma$SL=SURFACE TENSION BETWEEN SOLID AND LIQUIS
$\gamma$LV=SURFACE TENSION BETWEEN LIQUID AND VAPOR

FIG. 2

FIG. 3

CAST LIQUID FILM        ADHESIVE POOL

FILM BACKING

COATING BARS

## FIG. 4

STAGE

HYDROPHILIC FILM

DROP

HORIZONTAL MEASURING
CROSS-LINE

ROTATE VERTICLE MEASURING
CROSS-LINE TO TANGENCY

VERTICLE MEASURING
CROSS-LINE

## FIG. 5

FIG. 6

FIG. 13

FIG. 14

FIG. 7

FIG. 8

EP 2 214 015 B2

FIG. 9

EP 2 214 015 B2

FIG. 10

47

49

51

51

53

45

## FIG. 11

55

45

49

57

## FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6048498 A
- US 6117395 A
- US 3686355 A
- US 5354815 A
- US 5614598 A
- US 5508313 A
- US 5660178 A
- US 6121508 A
- WO 0056828 A
- EP 869979 B
- US 5685758 A
- WO 9748779 A
- US 6040048 A
- US 2502841 A
- US 2829070 A
- US 3142582 A
- US 3326742 A
- US 3561995 A
- US 3843617 A
- US 3968309 A
- US 4190689 A
- US 4387183 A
- US 4416749 A
- US 4460652 A
- US 4595632 A
- US 4666452 A
- US 5273812 A
- US 5280084 A
- US 5332625 A
- US 5451460 A
- US 5503897 A
- US 5910287 A
- US 6171780 A

**Non-patent literature cited in the description**

- Glucose Monitor without Fingersticking. *IVD Technology,* July 1999, 16
- **WALTER J. MOORE.** Physical Chemistry. Prentice-Hall, 1962, 730
- Influence of Constitution on Adhesion. **W.A. ZISMAN.** Handbook of Adhesives. Van Nostrand Reinhold Co, 1977, 38
- **T. YOUNG.** *Philos. Trans. Roy. Soc.,* vol. 95 (1805), 65
- *The Physical Chemistry of Surface Films,* 1952
- **P.H. WINFIELD et al.** The Use of Flame Ionization Technology to Improve the Wettability and Adhesive Properties of Wood. *Int'l Journal of Adhesion and Adhesives,* 2001, vol. 21 (2
- **J.M. EVANS.** *J. Adhesion,* 1973, vol. 5, 1-7
- **J.M. EVANS.** *J. Adhesion,* 9-16
- **D.K. OWENS.** *J. App. Polymer Science,* 1975, vol. 19, 275-271, 3315-3326
- **D. BRIGGS et al.** *Journal Material Science,* vol. 11 (976
- **M.J. ROSEN.** Surfactant and Interfacial Phenomena. John Wiley & Sons, 1978
- **TH.F. TADROS.** Surfactants. Academic Press, Inc, 1984
- **A.C. CLARK et al.** New and Improved Waterborne Systems. *Adhesives Age,* September 1999, 33-40
- **A. DORING et al.** Atomic Force Microscopy: Micro- and Nano-Mapping of Adhesion, Tack and Viscosity. *Annual Technical Seminar: Pressure Sensitive Adhesive Tapes for the New Millennium,* May 2000, 213-222
- Influence of Constitution on Adhesion. **W.A. ZISMAN.** Handbook of Adhesives. 1977, 46
- Electronic Properties of Polymers. 1982, 177